# EUROPÄISCHE PATENTSCHRIFT

(11) **EP 1 515 658 B1**
(45) Veröffentlichungstag und Bekanntmachung des Hinweises auf die Patenterteilung: **08.11.2006**
(21) Anmeldenummer: 02751065.0
(22) Anmeldetag: 24.06.2002
(51) Int. Cl.: A61B 17/86, A61B 17/80

(54) **WIRBELSÄULENSTÜTZSYSTEM**
SPINAL COLUMN SUPPORT SYSTEM
SYSTEME DE SOUTIEN DE LA COLONNE VERTEBRALE

(43) Veröffentlichungstag der Anmeldung: 23.03.2005
(73) Patentinhaber: Brinkhaus, Bernhard, 8955 Oetwil an der Limmat (CH)
(72) Erfinder: Brinkhaus, Bernhard, 8955 Oetwil an der Limmat (CH)
(74) Vertreter: König, Beate
(86) Internationale Anmeldenummer: PCT/EP2002/006972
(87) Internationale Veröffentlichungsnummer: WO 2004/000147

(56) Entgegenhaltungen:
- WO-A-00/15125
- WO-A-02/00124
- WO-A-95/27444
- DE-A- 19 512 709
- FR-A- 2 763 828
- US-B1- 6 355 038

## Beschreibung

### Technisches Gebiet

Die vorliegende Erfindung bezieht sich auf das Gebiet von Wirbelsäulenstützsystemen. Diese dienen zur Behebung oder Linderung von Wirbelsäulenproblemen bzw. Wirbelsäulenkrankheiten. Genauer, die Erfindung betrifft ein System, bei welchem einzelne Wirbel entweder starr über ein Platten-/Schraubensystem fixiert werden oder zueinander in einem definierten Bewegungsraum eine eingeschränkte Restbeweglichkeit haben.

### Stand der Technik

Bei den heute auf dem Markt befindlichen Systemen werden die Wirbel fest über ein Stützsystem miteinander verbunden. Diese Systeme setzen immer eine gerade Linie aller in die einzelnen Wirbel verschraubter Schrauben voraus, damit die Verbindungsstangen oder Verbindungsplatten in diese integriert werden können. Dieses Ideal ist praktisch durch die Anordnung und Verschiedenheit der menschlichen Wirbel und der möglichen erzielbaren Genauigkeit durch den Chirurgen nicht gegeben. Nur durch einen, eigentlich in der Regel nicht gewollten Eingriff in die Biomechanik der Wirbel können die Schrauben in eine ausgerichtete Zwangslinie gebracht werden, indem verschiedene Wirbel mit der herausragenden Schraube gedreht werden. Somit werden permanente, nicht gewollte Verspannungen in das Wirbelsystem implantiert.

Bei den angewendeten Rundstangen ist zudem von der Geometrie her schon eine schlechte statische Stützfunktion gegeben. In der Industrie sind Rundstangen als Träger nicht zu finden.

Ein Beispiel eines bekannten Stützsystems mit fester Wirbelfixierung ist in der DE 195 10 543 C2 beschrieben. Eine Schraube weist an einem Ende einen Knochenschraubenschaft und und am anderen Ende einen Gewindeabschnitt auf, auf den eine mutterartige Grundplatte aufgeschraubt ist. Die Grundplatte weist an der dem Knochenschraubenschaft entgegengesetzten Oberfläche eine Rille auf, in die ein Fixierstab zum Verbinden mit einer weiteren derartigen Einrichtung einführbar ist.

Bei den heutigen Stangen- und Schlitzsystemen wird eine Schraube und der mit ihr fest verbundene Wirbel so gedreht, daß die Schraube ihre Unterbringung in dem engen Schlitz oder der Stangenlinie findet. Hierdurch ergibt sich für den Chirurgen keine einfache Ausgangssituation, weil er nicht weiß, welche Kräfte in das Wirbelsäulensystem gelangen und wie sie wirken. Der Patient kann aufgrunddessen permanente Schmerzen für lange Zeit haben.

Aus der WO 95/27444 A1 (Basis für den Oberbegriff des Anspruchs 1) ist ein Wirbelsäulenstützsystem bekannt, umfassend eine langgestreckte Plattenanordnung mit Längsschlitzen, in denen jeweils eine Knochenschraube verschiebbar angeordnet ist. Mit exzentrischen Öffnungen versehene halbkugelförmige Muttern sitzen auf der Knochenschraube ober- und unterhalb der Plattenanordnung und werden durch eine Gegenmutter mit der Plattenanordnung verspannt. Durch Verdrehung der halbkugelförmigen Muttern ist nur die Verkippung der Plattenanordnung einstellbar. Die halbkugelförmigen Muttern lassen sich nicht einfach fixieren, so daß das System keine große Verbreitung gefunden hat.

An ein taugliches und gutes biomechanisches, d.h. insbesondere an die menschliche Anatomie angepaßtes Wirbelstützsystem, und die im Operationssaal vorhandenen Möglichkeiten der Justierung und Ausrichtung der Wirbelschrauben, wird eine Reihe von sehr wichtigen Anforderungen gestellt:
- Die verwendeten Knochenschrauben müssen eine gute Ausreißfestigkeit aufweisen.
- Sie müssen später gegebenenfalls wieder herausnehmbar sein.
- Der Austrittsteil der Schraube muß so konstruiert sein, daß gewisse Schrägstellungen der Knochenschraube korrigierbar sind.
- Die Knochenschraube muß somit multiaxial sein.
- Am Übergang der Knochenschraube auf eine senkrechte Stützplatte muß die Schraube eine Beweglichkeit haben, damit der Wirbel im Bandscheibenbereich kleine kegelförmige Bewegungen ausführen kann.
- Die ideale Knochenschraube muß somit multiaxial sein und eine kleine kegelförmige Beweglichkeit haben.
- Das senkrechte Stützsystem muß ein Plattensystem sein, in dem nicht ein sehr schmaler Spalt für die Aufnahme der Schrauben zur Verfügung steht, sondern eine Zone, in der die Knochenschraube fest und senkrecht angeschraubt werden kann.
- Eine Feinjustage der Wirbelabstände vor dem definitiven Anziehen ist ebenfalls eine große Hilfe für den Chirugen und Patienten.

Die heute bekannten Systeme werden diesen gesamten Anforderungen nicht gerecht, wobei die komplizierten anatomischen Verhältnisse und das nicht machbare exakte Setzen der Knochenwirbelschrauben die entscheidenen Faktoren sind.

### Darstellung der Erfindung

Es ist daher die Aufgabe der Erfindung, ein Wirbelstützsystem zu schaffen, das diese Schwierigkeiten überwindet und die negativen Einflußfaktoren auf das Wirbelsäulensystem beseitigt und den Heilungsprozeß neben der reinen Stützfunktion positiv beeinflußt.

Diese Aufgabe ist bei einem Wirbelsäulenstützsystem mit den Merkmalen des Anspruchs 1 gelöst. Vorteilhafte Weiterbildungen des erfindungsgemäßen Wirbelsäulenstützsystems sind Gegenstand der abhängigen Ansprüche.

Ein Wirbelsäulenstützsystem gemäß der Erfindung umfaßt somit eine oberhalb des Knochenschraubenschafts axial beweglich ausgebildete Knochenschraube, eine Platten- oder Stangenanordnung mit mindestens einer Öffnung, in der die Knochenschraube verschraubt ist, sowie eine obere und eine untere Scheibe, die in der Platte oder Stange beabstandet übereinander verschiebbar positionierbar aufgenommen sind und die jeweils ein Loch aufweisen, durch das die Knochenschraube durchgeführt ist.

Dabei sind die obere und/oder untere Scheibe mit exzentrisch, d.h. außerhalb der Mittenachse der Scheiben, angeordneten Löchern, z.B. einer kreisförmigen Bohrung, versehen und drehbar angebracht. Hierzu werden zweckmäßig kreisförmige-Scheiben in die Einheiten auf der Ober- und Unterseite der Platte oder Stange eingelegt und frei fixiert.

Die untere Scheibe enthält vorteilhaft eine kegelförmige Bohrung, die sich nicht in ihrer Mittelachse befindet.

Durch die freie Beweglichkeit der Scheiben und durch Drehung dieser selbst können die Bohrungen der oberen und unteren Scheiben senkrecht übereinander so fixiert werden, daß sich der obere Teil der Knochenschraube senkrecht einführen läßt.

Infolge der axial beweglichen Ausbildung der Knochenschraube oberhalb des Knochenschraubenschafts läßt sich die Knochenschraube durch Drehen oder Neigen immer senkrecht im Platten-oder Stangensystem befestigen, wenn sich der Knochenschraubenschaft in einer nicht genau definierten Position im Wirbel des Patienten befindet. Auf diese Weise können die Knochenschrauben die Wirbelknochen spannungs- und kräftefrei untereinander stabilisieren.

Ein erfindungsgemäßes Wirbelstützsystem ist somit als Platte mit geeigneten Öffnungen und Querstabilisatoren ausgebildet, damit die zu fixierenden Knochenschrauben, egal wie sie aus dem Wirbelknochen ragen, mit Hilfe von zwei kreisrunden Scheiben, in denen sich exzentrische Rundlöcher befinden, fest geschraubt werden können. Die Knochenschrauben selbst sind zudem im oberen Teil multiaxial ausgebildet und erlauben somit extreme Schrägstellungen der Wirbelknochenschrauben.

Die Plattenöffnungen sind zweckmäßig so ausgeführt, daß sie aus einem Rechteck oder Quadrat mit jeweils an den kurzen Seiten angesetzten Halbkreisen besteht (Langloch), die sich an der Unterseite und an der Oberseite der Platte befinden.

Bei einem vorteilhaften Ausführungsbeispiel des erfindungsgemäßen Wirbelsäulenstützsystems sind die Knochenschrauben multiaxial ausgebildet und eine den oberen Teil der Knochenschraube bildende Klemmschraube ist über ein Kugelsystem mit der Knochenschraube gekoppelt. Die Klemmschraube wird immer senkrecht in das Plattensystem eingeführt, wobei die Einführung der Klemmschraube durch freie Fixierung der oberen und unteren kreisförmigen Scheiben kräftefrei erfolgen kann. Durch Festziehen der Muttern kann das ganze System kräftefrei an die zu stützenden und zu fixierenden Wirbel angekoppelt bzw. angepaßt werden.

Eine zweite Art von Knochenschrauben ist praktisch identisch, gibt aber im eingeschraubten Zustand dem Wirbelknochen noch eine definierte Restbeweglichkeit.

Anders als bei den heutigen, am Markt befindlichen Systemen, bei denen die Knochenschrauben immer in eine linienförmige exakte Ausrichtung in die Wirbelknochen geschraubt werden müssen, damit eine Verschraubung über ein Stangensystem oder eine schmale schlitzartige Platte überhaupt möglich ist, wird durch die erfindungsgemäße Platte und deren zugehörige Schrauben sichergestellt, daß auch bei extremen Austrittsstellungen der Wirbelknochenschrauben diese ohne auch nur die geringste zusätzliche Kraftanwendung in die Platte festgeschraubt werden können.

Eine bevorzugte Ausführungsform der Erfindung zeichnet sich dadurch aus, daß sich die Stützplatte aus modular identischen Funktionseinheiten zusammensetzt. Das heißt, die Platte kann nach Anzahl der zu stützenden Wirbel durch identische Einheiten verlängert werden. Dies ergibt eine sehr stabile Form mit definiert federnd wirkenden Zonen. Die Plattenelemente sind so gestaltet, daß die Öffnungsform mit ihrem innen liegenden Stützring die obere runde Scheibe und die untere runde dickere Scheibe aufnehmen und in ihr beliebig verschiebbar und positionierbar sind. Durch Drehen der unteren Scheibe (anschließend der oberen Scheibe) kann das in ihr befindliche konische Loch zum Einführen der Klemmschraube frei positioniert werden. Mit dem Anziehen der Mutter werden sowohl der Kugelkopf der Knochenschraube, als auch die obere Platte und die untere Platte am innen liegenden Stützring fest geklemmt. Die untere und die obere Scheibe sind dann nicht mehr verschiebbar. Ebenfalls ist so die Knochenschraube fixiert, wobei durch das Hineinziehen der Klemmschraube in die kegelförmige Bohrung der unteren Scheibe das kugelförmige Koppelelement zwischen Knochenschraube und Klemmschraube blockiert wird.

Der Chirurg hat demgemäß die Möglichkeit, die gesetzten Knochenschrauben ohne Einwirkung von Kräften auf das Wirbelsystem in dem Plattensystem zu fixieren.

Eine weitere bevorzugte Ausführungsform der erfindungsgemäßen Stabilisierung ist das sogenannte semirigide Wirbelsäulenstützsystem. Dieses besteht darin, daß die Knochenschrauben direkt hinter dem multiaxialen Gelenk noch ein Kalottensystem angeordnet haben, das dem vorderen und damit dem in den Knochen geschraubten Teil der Knochenschraube eine kegel- oder pyramidenförmige freie Bewegung erlaubt. Diese kleine Bewegungsfreiheit der gestützten Wirbel regt die Durchblutung positiv für den Heilungsprozeß an. Das kalottenförmige Gelenk ist idealerweise direkt am Wirbelgelenk angeordnet und hält dieses in einem eingeschränktem Bereich beweglich. Vorzugsweise enthält das kalottenförmige Element gleichzeitig eine gestufte Verdrehsicherung.

### Kurze Erläuterungen der Zeichnungen

- Fig. 1: zeigt ein erstes Ausführungsbeispiel eines Wirbelsäulenstützsystems gemäß der Erfindung in rigider Ausführung in der Draufsicht;
- Fig. 2: zeigt eine Seitenansicht des Wirbelsäulenstützsystems von Fig. 1;
- Fig. 3: zeigt ein zweites Ausführungsbeispiels eines Wirbelsäulenstützsystems gemäß der Erfindung in semirigider Ausführung in der Draufsicht; und
- Fig. 4: zeigt eine Seitenansicht des Wirbelsäulenstützsystems von Fig. 3.

### Beschreibung bevorzugter Ausführungsbeispiele

Die Erfindung wird im folgenden anhand von zwei Ausführungsbeispielen mehr im einzelnen erläutert. Diese Darstellung soll die Erfindung jedoch nicht auf die konkret beschriebenen Merkmalskombinationen beschränken, so wie auch die Erfindung nicht auf die in den abhängigen Ansprüchen angegebenen Merkmalskombinationen beschränkt sein soll.

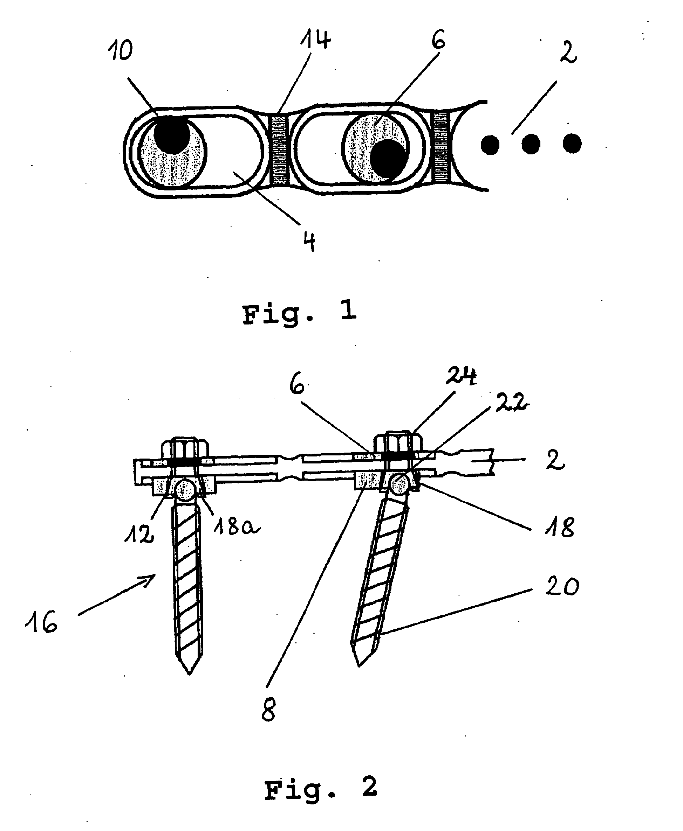

Es wird zunächst auf Fig. 1 und 2 Bezug genommen, die ein erstes Ausführungsbeispiel in Form eines rigiden Wirbelsäulenstützsystems zeigen. Eine Platte 2 weist eine langgestreckte Öffnung 4 auf. In der Öffnung 4 sind übereinander mit Abstand angeordnet zwei kreisrunde Scheiben, eine obere Scheibe 6 und eine untere dickere Scheibe 8, längsverschiebbar und drehbar aufgenommen. Die Scheiben 6, 8 weisen jeweils exzentrisch angeordnete Löcher 10, 12 auf, von denen das untere konisch ausgeführt ist, d.h. sich nach unten erweitert. Zwischen derartigen Einheiten der Platte 2 befinden sich federnde Zonen, die als Biegestelle 14 ausgebildet sind.

Durch die Löcher 10, 12 ist eine Knochenschraube 16 mit einem oberen Teil 18 und einem in bezug auf diesen beweglich ausgebildeten unteren Teil 20 geführt und im Bereich des oberen Teils fixiert, d.h. festgeschraubt. Der obere Teil der Knochenschraube 16 ist im veranschaulichten Ausführungsbeispiel als Klemmschraube 18 mit Klemmkörper 18a ausgeführt. Der untere Teil der Knochenschraube 16 ist der eigentliche Knochenschraubenschaft 20. Am oberen Ende ist der Knochenschraubenschaft mit einem Kugelkopf 22 versehen, der drehbeweglich im Klemmkörper 18a aufgenommen ist. Zur Fixierung der Anordnung dient eine Mutter 24, die zum einen den Kugelkopf 22 im Klemmkörper 18a und zum anderen die beiden Scheiben 6, 8 in der Platte 4 fest klemmt.

Durch Verschieben und Drehen der beiden Scheiben 6, 8 zur Ausrichtung des Wirbelsäulenstützsystems nach dem Einbringen der Knochenschrauben in die Wirbelknochen können Schrägstellungen der Knochenschraube, d.h. des Knochenschraubenschafts 20, ausgeglichen werden. Eine weitere Flexibilität der Anordnung liefert die Multiaxialität der Knochenschraube 16 mit ihren beiden Teilen 18, 20.

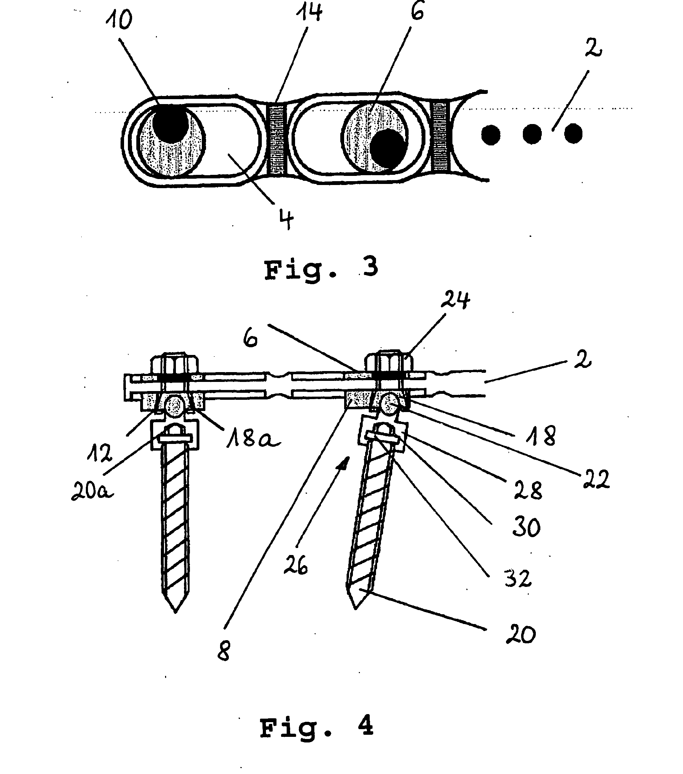

Anhand von Fig. 3 und 4 wird ein zweites Ausführungsbeispiel der Erfindung in Form eines semirigiden Wirbelsäulenstützsystems beschrieben. Soweit die Teile dieselben wie diejenigen des ersten Ausführungsbeispiels sind, werden sie nicht erneut beschrieben.

Bei diesem Ausführungsbeispiel endet der Knochenschraubenschaft 20 nicht direkt in dem Kugelkopf 22, sondern es ist eine wiederum beweglich angeordnete, als Kalottensystem ausgeführte Halterung (Halterungselement) 26 zusätzlich vorgesehen. Die Halterung 26 endet an ihrem oberen Ende im Kugelkopf 22 und besteht aus einem Kalottenkörper 28, der auf der Unterseite ein Kalottenlager 30 aufweist. In dem Kalottenlager 30 ist das obere Ende 20a des Knochenschraubenschafts 20 kegel-oder pyramidenförmig frei beweglich gelagert. Eine stufig ausgebildete Verdrehsicherung 32 ist im Kalottenkörper 28 vorgesehen.

Durch die so zusätzlich in geringem Umfang ermöglichte Bewegungsfreiheit der gestützten Wirbel wird die Durchblutung angeregt und der Heilprozeß beschleunigt.

## Patentansprüche

1. Wirbelsäulenstützsystem, umfassend eine Knochenschraube (16), eine Platten- oder Stangenanordnung mit mindestens einer Öffnung (4), in der die Knochenschraube (16) verschiebbar ist, ein oberes und ein unteres drehbares Fixierelement, die bezüglich der Platte (2) oder Stange beabstandet übereinander positionierbar sind und die jeweils ein exzentrisch angeordnetes Loch (10, 12) aufweisen, durch das die Knochenschraube (16) durchgeführt ist, **dadurch gekennzeichnet , daß** die Knochenschraube (16) oberhalb des Knochenschraubenschafts axial beweglich ausgebildet ist und als Fixierelemente jeweils eine obere und eine untere Scheibe (6, 8) vorgesehen sind, die in der Platte (2) oder Stange verschiebbar aufgenommen sind und jeweils ein exzentrisch angeordnetes Loch (10, 12) aufweisen.

2. Wirbelsäulenstützsystem nach Anspruch 1, **dadurch gekennzeichnet, daß** die Platte oder Stange in der Innenwand der Öffnung mit einem Stützring versehen ist, der die obere und die untere Scheibe aufnimmt.

3. Wirbelsäulenstützsystem nach einem Anspruch 1 oder 2, **dadurch gekennzeichnet , daß** die obere und die untere Scheibe (6, 8) kreisförmig sind.

4. Wirbelsäulenstützsystem nach einem der Ansprüche 1 bis 3, **dadurch gekennzeichnet , daß** die untere Scheibe (8) eine größere Dicke als die obere Scheibe (6) aufweist.

5. Wirbelsäulenstützsystem nach einem der Ansprüche 1 bis 4, **dadurch gekennzeichnet , daß** das Loch (12) der unteren Scheibe (8) konisch ist.

6. Wirbelsäulenstützsystem nach einem der Ansprüche 1 bis 5, ddurch **gekennzeichnet** , daß die Knochenschraube (16) aus einem zur Verschraubung in der Platte (2) oder Stange vorgesehenen oberen Teil (18), der am unteren Ende eine kugelförmige Aufnahme aufweist, und einem unteren Knochenschraubenschaftteil (20) besteht, der am oberen Ende einen in der kugelförmigen Aufnahme drehbeweglich aufgenommenen Kugelkopf (22) aufweist.

7. Wirbelsäulenstützsystem nach Anspruch 6, **dadurch gekennzeichnet, daß** der Knochenschraubenschaftteil (20) am oberen Ende mit einem Halterungselement (26) versehen ist, das am oberen Ende den drehbeweglich im oberen Knochenschraubenteil aufgenommenen Kugelkopf (22) und unterhalb desselben ein Kalottenlager (30) zur Aufnahme des oberen Endes (20a) des Knochenschraubenschaftteils (20) aufweist derart, daß das letztere in dem Kalottenlager kegel- oder pyramidenförmig frei beweglich ist.

8. Wirbelsäulenstützsystem nach Anspruch 7, **dadurch gekennzeichnet, daß** in dem Kalottenlager (30) eine stufig ausgebildete Verdrehsicherung (32) vorgesehen ist.

9. Wirbelsäulenstützsystem nach einem der Ansprüche 1 bis 8, **dadurch gekennzeichnet , daß** der obere Teil der Knochenschraube eine Klemmschraube (18) ist, die an ihrem zu dem Knochenschraubenschaft (20) entgegengesetzten Ende gewindeversehen ist und mit einer Mutter (24) fixierbar ist.

10. Wirbelsäulenstützsystem nach einem der Ansprüche 1 bis 9, **dadurch gekennzeichnet , daß** ein oder mehrere Einheiten als Verlängerung zum Stützen eines oder mehrerer Wirbel vorgesehen sind.

11. Wirbelsäulenstützsystem nach Anspruch 10, **dadurch gekennzeichnet, daß** zwischen benachbarten Einheiten eine Biegezone (14) vorgesehen ist.

## Claims

1. Spinal column support system comprising a bone screw (16), a plate or rod arrangement having at least one opening (4) in which the bone screw (16) can be displaced, an upper and a lower rotatable fixing element which can be positioned one above the other at a spaced disposition relative to the plate (2) or rod and each comprise an eccentrically disposed hole (10, 12) through which the bone screw (16) is passed, **characterised in that** the bone screw (16) is formed so as to be movable in an axial manner above the bone screw shaft and an upper and a lower disc (6, 8) are respectively provided as the fixing elements, are received in a displaceable manner in the plate (2) or rod and each comprise an eccentrically disposed hole (10, 12).

2. Spinal column support system as claimed in Claim 1, **characterised in that** the plate or rod is provided with a support ring in the inner wall of the opening, said support ring receiving the upper and the lower discs.

3. Spinal column support system as claimed in any one of Claims 1 or 2, **characterised in that** the upper and the lower discs (6, 8) are circular.

4. Spinal column support system as claimed in any one of Claims 1 to 3, **characterised in that** the lower disc (8) is thicker than the upper disc (6).

5. Spinal column support system as claimed in any one of Claims 1 to 4, **characterised in that** the hole (12) of the lower disc (8) is conical.

6. Spinal column support system as claimed in any one of Claims 1 to 5, **characterised in that** the bone screw (16) consists of an upper part (18), which is provided for screwing in the plate (2) or rod and has a spherical recess at the lower end, and of a lower bone screw shaft part (20) which has at the upper end a spherical head (22) received in the spherical recess in a rotationally-movable manner.

7. Spinal column support system as claimed in Claim 6, **characterised in that** the bone screw shaft part (20) is provided at the upper end with a retaining element (26) which comprises at the upper end the spherical head (22) received in the upper bone screw part in a rotationally-movable manner and comprises beneath the spherical 5 head a cup and ball bearing (30) for receiving the upper end (20a) of the bone screw shaft part (20) such that the upper end is freely movable in the cup and ball bearing in a cone- or pyramid-like manner.

8. Spinal column support system as claimed in Claim 7, **characterised in that** a lock against rotation (32) formed in a step-like manner is provided in the cup and ball bearing (30).

9. Spinal column support system as claimed in any one of Claims 1 to 8, **characterised in that** the upper part of the bone screw is a clamping screw (18) which is provided with a thread at its end opposite the bone screw shaft (20) and can be fixed to a nut (24).

10. Spinal column support system as claimed in any one of Claims 1 to 9, **characterised in that** one or several units are provided as an extension to support one or several vertebrae.

11. Spinal column support system as claimed in Claim 10, **characterised in that** a bending zone (14) is provided between adjacent units.

## Revendications

1. Dispositif de soutien de la colonne vertébrale comprenant une vis à os (16), un agencement de plaques ou de tiges avec au moins une ouverture (4) dans laquelle la vis à os (16) peut coulisser, un élément de fixation supérieur et un élément de fixation inférieur mobiles en rotation qui, vis-à-vis de la plaque (2) ou de la tige, peuvent être positionnés l'un au-dessus de l'autre à distance l'un de l'autre et qui présentent chacun un trou (10, 12) excentré dans lequel passe la vis à os (16), **caractérisé par le fait que** la vis à os (16), au-dessus de la tige de vis à os, est mobile axialement et qu'il est prévu comme éléments de blocage respectivement une rondelle supérieure et une rondelle inférieure (6, 8), lesquelles rondelles sont reçues de manière coulissante dans la plaque (2) ou la tige et présentent chacune un trou (10, 12) excentré.

2. Dispositif de soutien de la colonne vertébrale selon la revendication 1, **caractérisé par le fait que** la plaque ou la tige, dans la paroi intérieure de l'ouverture, est pourvue d'un appui annulaire qui reçoit la rondelle supérieure et la rondelle inférieure.

3. Dispositif de soutien de la colonne vertébrale selon la revendication 1 ou 2, **caractérisé par le fait que** la rondelle supérieure et la rondelle inférieure (6, 8) sont circulaires.

4. Dispositif de soutien de la colonne vertébrale selon une des revendications 1 à 3, **caractérisé par le fait que** la rondelle inférieure (8) présente une épaisseur supérieure à celle de la rondelle supérieure (6).

5. Dispositif de soutien de la colonne vertébrale selon une des revendications 1 à 4, **caractérisé par le fait que** le trou (12) de la rondelle inférieure (8) est conique.

6. Dispositif de soutien de la colonne vertébrale selon une des revendications 1 à 5, **caractérisé par le fait que** la vis à os (16) comprend une partie supérieure (18) qui est prévue pour être vissée dans la plaque (2) ou la tige et dont l'extrémité inférieure présente un logement sphérique, et une partie tige de vis à os (20) inférieure qui présente à son extrémité supérieure une tête sphérique (22) montée tournante dans le logement sphérique.

7. Dispositif de soutien de la colonne vertébrale selon la revendication 6, **caractérisé par le fait que** la partie tige de vis à os (20), à son extrémité supérieure, est pourvue d'un élément de retenue (26) qui présente à l'extrémité supérieure la tête sphérique (22) montée tournante dans la partie de vis à os supérieure et présente au-dessous de celle-ci un palier en forme de calotte (30) pour recevoir l'extrémité supérieure (20a) de la partie tige de vis à os (20) inférieure, tel que cette dernière puisse se déplacer librement suivant un cône ou une pyramide dans le palier sphérique.

8. Dispositif de soutien de la colonne vertébrale selon la revendication 7, **caractérisé par le fait qu'**il est prévu dans le palier sphérique (30) un moyen de freinage en rotation (32) conformé en gradin.

9. Dispositif de soutien de la colonne vertébrale selon une des revendications 1 à 8, **caractérisé par le fait que** la partie supérieure de la vis à os est une vis de serrage (18) dont l'extrémité opposée à la tige de vis à os (20) est munie d'un filetage et qui peut être bloquée au moyen d'un écrou (24).

10. Dispositif de soutien de la colonne vertébrale selon une des revendications 1 à 9, **caractérisé par le fait qu'**une ou plusieurs unités sont prévues comme extension pour le soutien d'une ou de plusieurs vertèbres.

11. Dispositif de soutien de la colonne vertébrale selon la revendication 10, **caractérisé par le fait qu'**une zone de flexion (14) est prévue entre deux unités voisines.
